# EUROPEAN PATENT SPECIFICATION

(11) **EP 3 638 259 B1**
(45) Date of publication and mention of the grant of the patent: **15.02.2023**
(21) Application number: 18737995.3
(22) Date of filing: 15.06.2018
(51) Int. Cl.: A61K 35/16, A61K 38/18, A61P 27/06, A61P 27/02, A61P 25/00

(54) **BLOOD SERUM FOR USE IN THE TREATMENT OF NEURODEGENERATIVE OPHTHALMOLOGICAL PATHOLOGIES**
BLUTSERUM ZUR VERWENDUNG BEI DER BEHANDLUNG VON NEURODEGENERATIVEN OPHTHALMOLOGISCHEN PATHOLOGIEN
SERUM DE SANG UTILISE DANS LE TRAITEMENT DE PATHOLOGIES OPHTALMOLOGIQUES NEURODEGENERATIVES

(30) Priority: 15.06.2017 IT 201700066486
(43) Date of publication of application: 22.04.2020
(73) Proprietor: Azienda Ospedaliero-universitaria Policlinico S. Orsola Malpighi-Bologna, 40138 Bologna (IT); Alma Mater Studiorum - Università di Bologna, 40126 Bologna (IT); Universita' degli Studi dell'Aquila, 67100 L'Aquila (IT)
(72) Inventor: CAMPOS, Emilio C., 40138 Bologna (IT); VERSURA, Piera, 40137 Bologna (IT); BUZZI, Marina, 40125 Bologna (IT); BISTI, Silvia, 16124 Genova (IT); VELATI, Claudio, 40122 Bologna (IT)
(74) Representative: Biggi, Cristina
(86) International application number: PCT/IB2018/054410
(87) International publication number: WO 2018/229718

(56) References cited:
- US-A- 5 210 185
- US-A1- 2007 014 760
- KOHSAKA S ET AL: "Increased activity of ornithine decarboxylase in goldfish following optic nerve crush", DEVELOPMENTAL BRAIN RESEARCH, ELSEVIER SCIENCE BV, AMSTERDAM, NL, vol. 1, no. 3, 1 June 1981 (1981-06-01), pages 391-401, XP024320502, ISSN: 0165-3806, DOI: 10.1016/0165-3806(81)90076-6 [retrieved on 1981-06-01]
- YIP H K ET AL: "Effect of nerve growth factor on regeneration of goldfish optic axons", BRAIN RESEARCH, ELSEVIER, AMSTERDAM, NL, vol. 238, no. 2, 29 April 1982 (1982-04-29), pages 329-339, XP024259227, ISSN: 0006-8993, DOI: 10.1016/0006-8993(82)90108-1 [retrieved on 1982-04-29]
- LAKSHMANAN J: "Beta-nerve growth factor measurements in mouse serum.", JOURNAL OF NEUROCHEMISTRY MAR 1986, vol. 46, no. 3, March 1986 (1986-03), pages 882-891, XP002777951, ISSN: 0022-3042
- AJAMI A ET AL: "Changes in serum levels of brain derived neurotrophic factor and nerve growth factor-beta in schizophrenic patients before and after treatment.", SCANDINAVIAN JOURNAL OF IMMUNOLOGY JUL 2014, vol. 80, no. 1, July 2014 (2014-07), pages 36-42, XP002777952, ISSN: 1365-3083
- Kyung Chul Yoon: "Use of Umbilical Cord Serum in Ophthalmology", Chonnam Medical Journal, vol. 50, no. 3, 1 January 2014 (2014-01-01), page 82, XP055190630, ISSN: 2233-7385, DOI: 10.4068/cmj.2014.50.3.82

## Description

### TECHNICAL FIELD

The present invention relates to the use of blood serum, preferably serum from umbilical cord blood, for the treatment of neurodegenerative ophthalmologic pathologies, in particular degenerative pathologies of the retina and of the optic nerve, such as glaucoma, and ophthalmologic pathologies whose progression is linked to the activation of neuroinflammatory processes. The serum for use according to the present invention comprises the nerve growth factor-β (NGF-β), preferably in a concentration equal to or greater than 5 pg/ml.

The invention also relates to a composition in the form of a collyrium, eye drops, eye ointment or eye gel which comprises the serum of the invention.

### BACKGROUND OF THE INVENTION

Neurodegenerative ophthalmologic pathologies, in particular degenerative pathologies of the retina, degenerative pathologies of the optic nerve and ophthalmologic pathologies whose progression is linked to the activation of neuroinflammatory processes, cause a progressive and disabling impairment of visual function, which is accompanied by a drastic reduction in the quality of life of the individuals who are affected by them, understood in the broadest social and economic sense. One of the most widespread and disabling degenerative ophthalmologic pathologies is glaucoma. The term "glaucoma" generally refers to a set of ocular pathologies which have in common the presence of chronic progressive damage of the optic nerve, with characteristic alterations in the appearance of the optic nerve itself and in the layer of retinal nerve fibres. Glaucoma can cause irreversible damage to eyesight and, in some cases, low vision or blindness. In the more advanced stages, the visual field of a patient with glaucoma is reduced to the central area only, whereas peripheral vision is compromised. The "visual field" is defined as the portion of space perceived by an eye fixated in a straight-ahead gaze. In the absence of alterations, a so-called "normal" visual field extends beyond 90° temporally, 60° nasally and superiorly and about 70° inferiorly. In the presence of alterations, these ranges are progressively and selectively reduced in the four quadrants.

The expression "neuroprotection" generally refers to all interventions aimed at reducing both early and late damage to neuronal cells following tissue damage, as occurs in many neurodegenerative pathologies. Currently ongoing studies (prevalently oriented towards neurodegenerative diseases such as Parkinson's disease and amyotrophic lateral sclerosis) have demonstrated the therapeutic effectiveness of the administration of specific neurotrophic factors whose levels are reduced in the pathology in question (or factors that induce the endogenous synthesis of such specific neurotrophic factors). As regards neurodegenerative pathologies in the ophthalmologic field, the neuroprotection treatments presently proposed are prevalently based on the administration of substances of natural origin such as amino acids and/or vitamins (of whose effectiveness there is no evidence, which is in any case not required at the stage of registration for placement on the market), or other compounds of vegetable origin (curcumin, flavonoids, LBP polysaccharides contained in goji berries, etc.). Such substances of natural origin can be taken directly through the diet (for example by increasing the consumption of fruit such as apples and oranges) or through dietary or nutraceutical supplements. An alternative therapeutic strategy is based on the administration of synthetic or extracted molecules (such as memantine, citicoline, brimonidine, homotaurine, polyphenols). In any case, clinical studies regarding neuroprotection in neurodegenerative pathologies in the field of ophthalmology are at the initial stages and there are not yet any available data confirming the clinical validity of these therapeutic approaches.

Therefore, despite the clinical progress of recent years, the possibility of developing new strategies for treating neurodegenerative pathologies of the retina and of the optic nerve, such as glaucoma, as well as ophthalmologic pathologies whose progression is linked to the activation of neuroinflammatory processes, is of great interest. In particular, there is a strong need to develop more effective therapeutic strategies not only in slowing down the progression of the pathology (so as to contain the functional damage and hence the visual impairment) but also in enabling a regression of the symptoms and clinical manifestations, with a lasting effect that is perceivable by the patient. US2007/014760, Kohsaka et al. Development Brain Research, vol. 1, no. 3, 1981, p. 391-402 and Yip et al., Brain Research, vol. 238, no. 2, 1982, p. 329-339 refer to the use of NGF-β to regenerate ocular nerve.

### SUMMARY OF THE INVENTION

The present invention relates to the blood serum for use in the treatment of neurodegenerative pathologies in the ophthalmologic field, selected from degenerative pathologies of the retina or of the optic nerve and ophthalmologic pathologies whose progression is linked to the activation of neuroinflammatory processes. The blood serum used comprises the nerve growth factor-β (NGF-β), preferably in a concentration equal to or greater than 5 pg/ml.

In one embodiment, the serum comprises the factor NGF-β in a concentration equal to or less than 5 pg/ml.

In one embodiment of the invention, the serum of the invention comprises the brain-derived neurotrophic factor (BDNF), preferably in a concentration equal to or greater than 9500 pg/ml.

The blood serum can preferably be serum from umbilical cord blood. The serum of the invention can be used as such or within a composition for the treatment of degenerative pathologies of the retina or of the optic nerve, in particular glaucoma.

The serum of the invention can be used as such or within a composition for the treatment of ophthalmologic pathologies whose progression is linked to the activation of neuroinflammatory processes, such as, for example, retinitis pigmentosa, age-related macular degeneration (AMD), diabetic retinopathies or Usher syndrome.

Therefore, the invention also relates to a composition which comprises the blood serum mentioned above and optionally one or more excipients and/or carriers and/or preservatives.

Furthermore, the invention relates to a method for preparing blood serum for the use mentioned above, so that it can be used in the treatment of neurodegenerative ophthalmologic pathologies.

### BRIEF DESCRIPTION OF THE FIGURES

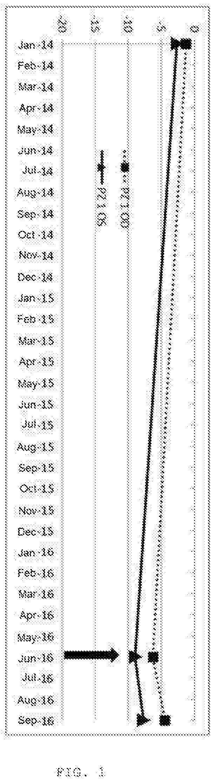

Figure 1 is a graph that shows the pattern in the MD (mean deviation) values of sensitivity in the visual field of a patient (PZ 1) affected by primary open-angle glaucoma (POAG), before and after treatment with the collyrium of the invention. The arrow corresponding to June 2016 indicates the start of the treatment with the serum. OD=right eye; OS=left eye.

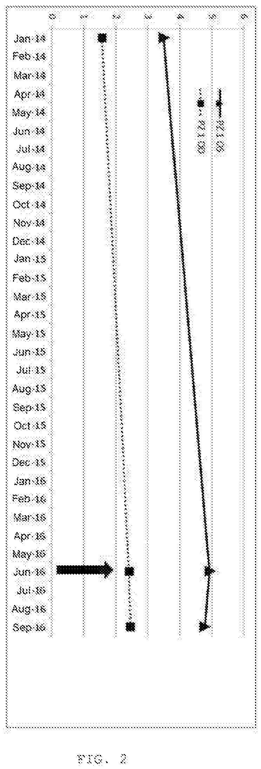

Figure 2 is a graph that shows the pattern in the PSD (pattern standard deviation) values of sensitivity in the visual field of a patient (PZ 1) affected by primary open-angle glaucoma (POAG), before and after treatment with the collyrium of the invention. The arrow corresponding to June 2016 indicates the start of the treatment with the serum. OD=right eye; OS=left eye.

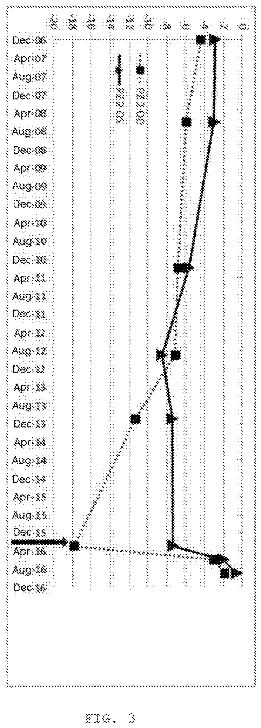

Figure 3 is a graph that shows the pattern in the MD (mean deviation) values of sensitivity in the visual field of a patient (PZ 2) affected by primary open-angle glaucoma (POAG), before and after treatment with the collyrium of the invention. The arrow corresponding to March 2016 indicates the start of the treatment with the serum. The serum was administered in the right eye only. OD=right eye; OS=left eye.

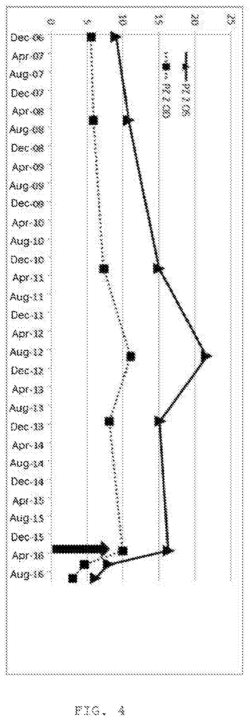

Figure 4 is a graph that shows the pattern in the PSD (pattern standard deviation) values of sensitivity in the visual field of a patient (PZ 2) affected by primary open-angle glaucoma (POAG), before and after the treatment with the serum collyrium of the invention. The arrow corresponding to March 2016 indicates the start of the treatment with the serum. The serum was administered in the right eye only. OD=right eye; OS=left eye.

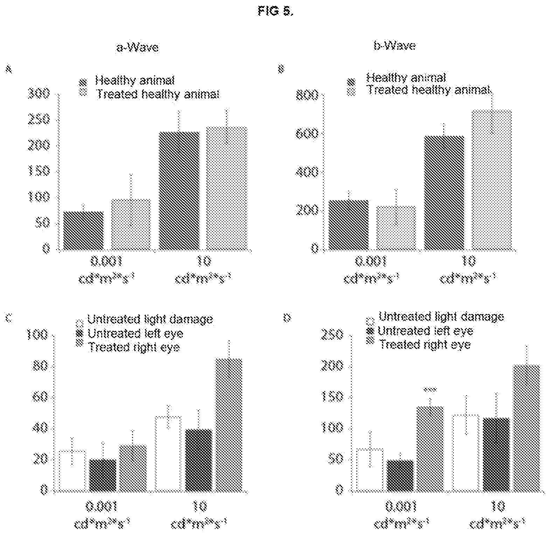

Figure 5 is an electroretinogram (fERG) recorded in response to flashes of increasing luminance, and which shows the amplitude of the a-wave and b-wave recorded at two different intensities in relation to: stimulation of the eye in a control animal treated and not treated with the serum of the invention (A and B); stimulation of the control eye damaged following exposure to light (LD control) and stimulation of the treated and untreated eye in the same animal exposed to light (LD treated eye and untreated eye)(C and D)

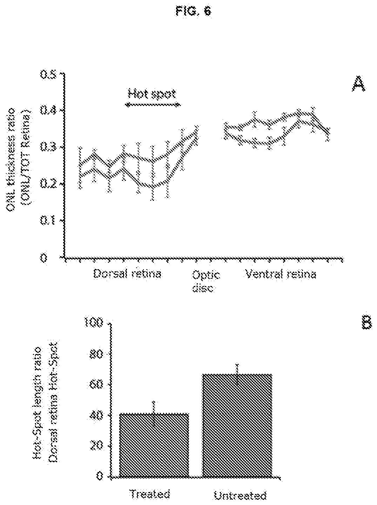

Figure 6A shows the thickness of the photoreceptor layer (ONL), measured from the dorsal to ventral retina which passes through the optic disc in a sample of a treated eye (upper line) and a sample of an untreated eye (lower line) both exposed to light LD.
Figure 6B shows the extent of the "hot spot" (area of maximum damage) in a sample of a treated eye (left) and an untreated eye (right).

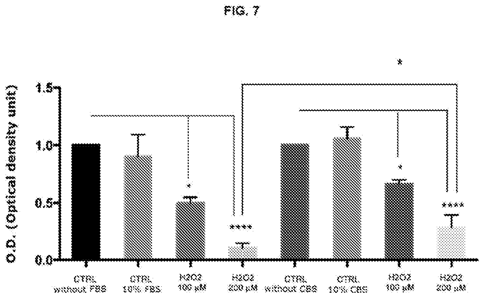

Figure 7 shows the analysis of cell viability in RMC-1 cells after exposure to H₂O₂ in the presence of FBS/CBS. The graph shows the differences in terms of cell viability after treatment with H₂O₂ between the RMC-1 cells cultured with FBS or CBS. The asterisks in the figure indicate, respectively, *: p<0.05; ***: p<0.01; ****p<0.0001.

### DETAILED DESCRIPTION OF THE INVENTION

The present invention relates to blood serum, comprising the nerve growth factor-β (NGF-β), preferably in a concentration equal to or greater than 5 pg/ml, for use in the treatment of neurodegenerative ophthalmologic pathologies, in particular degenerative pathologies of the retina or of the optic nerve or for use in the treatment of ophthalmologic pathologies whose progression is linked to the activation of neuroinflammatory processes.

In one embodiment, the blood serum for use according to the invention can comprise NGF-β in a concentration equal to or greater than 6 pg/ml; more preferably, the blood serum for use according to the invention can comprise NGF-β in a concentration equal to or greater than 7 pg/ml; even more preferably, the blood serum for use according to the invention can comprise NGF-β in a concentration equal to or greater than 8 pg/ml.

In one embodiment, the factor NGF-β is present in the serum in a concentration equal to or less than 5 pg/ml, preferably comprised between 0.5 and 5 pg/ml, advantageously comprised between 0.7 and 4 pg/ml.

The nerve growth factor-β (NGF-β) is a powerful neurotrophic factor (belonging to the family of neurotrophins) which supports the growth and survival of nerve cells and glia. NGF-β, in combination with the other factors present in serum, is responsible for the neuroprotective and anti-inflammatory effect of the blood serum according to the invention.

The role of many growth factors and cytokines in cell growth, proliferation and differentiation has been known for some time; however, many cell communication pathways have not yet been fully understood and the way in which various routes of cell communication intersect with one another is still unknown. Therefore, in addition to NGF-β, various other molecules may play an important role in mediating a neuroprotective effect against ophthalmologic pathologies; among these molecules, it is possible to indicate growth factors, such as epidermal growth factor (EGF), transforming growth factor-α (TGF-α), platelet-derived growth factor (PDGF), fibroblast growth factor (FGF-basic), brain-derived neurotrophic factor (BDNF) and glial cell-line derived neurotrophic factor (GDNF).

Other factors that may play an important role in mediating a neuroprotective or anti-inflammatory effect against ophthalmologic pathologies, in combination with one or more of the other factors specified above, are TGF-β1, β2 and β3 (transforming growth factor-β1, β2 and β3), IGF-1 (insulin-like growth factor 1), IGF-2 (insulin-like growth factor 2) and VEGF (vascular endothelial growth factor).

Transforming growth factor-α (TGF-α) is produced by macrophages, brain cells and keratinocytes. TGF-α induces the development of the epithelium and its action is closely correlated to that of the epidermal growth factor (EGF), which performs an important role in regulating cell growth, proliferation and differentiation. TGF-α also stimulates the proliferation of neuronal cells in injured adult brains.

Platelet-derived growth factor (PDGF) is the principal serum mitogen for cells of mesenchymal origin. The biologically active protein is a dimer of two polypeptides (subunits A and B), which can exist in the form of a homodimer (AA or BB) and in the form of a heterodimer (AB).

Fibroblast growth factors (FGFs) are a family of growth factors involved in processes such as angiogenesis, wound healing and embryonic development and in various endocrine signalling pathways. During the development of the central nervous system, for example, FGFs play an important role in the proliferation of neuronal cells, neurogenesis, the development of axons and differentiation. FGF2 is produced and released by *Müller cells* (retinal glia) and is activated in self-protection mechanisms.

In the light of recent experimental evidence, BDNF seems to protect photoreceptors from the damage induced by light in an animal model, and GDNF seems to have the ability to protect retinal neurons

An indirect neuroprotective or anti-inflammatory role can also be played by some cytokines such as interleukins 10 and 13 (IL-10 and IL-13), which, by virtue of their anti-inflammatory action, contribute to hindering the neurodegenerative process. Interleukins IL-1 β, IL-4 and IL-6 can also have a neuroprotective or anti-inflammatory role.

In a preferred embodiment, therefore, the serum for use according to the present invention can further comprise, in addition to NGF-β, one or more growth factors or cytokines. In particular, the serum can comprise one or more molecules selected from the group consisting of:
- EGF, preferably in a concentration equal to or greater than 500 pg/ml;
- TGF-α, preferably in a concentration equal to or greater than 50 pg/ml;
- PDGF, preferably in a concentration equal to or greater than 5000 pg/ml;
- FGF, preferably in a concentration equal to or greater than 200 pg/ml;
- IL-10, preferably in a concentration equal to or greater than 5 pg/ml;
- IL-13, preferably in a concentration equal to or greater than 100 pg/ml;
- BDNF in a concentration equal to or greater than 9500 pg/ml;
- GDNF in a concentration equal to or greater than 0.5 pg/ml.

In one embodiment, EGF can be present in the serum for use according to the invention in a concentration equal to or greater than 700 pg/ml, preferably equal to or greater than 1000 pg/ml.

In one embodiment, EGF is present in the serum in a concentration of 500 to 1500 pg/ml, preferably 500 to 1200 pg/ml, advantageously 700 to 1000 pg/ml.

In one embodiment, TGF-α can be present in the serum for use according to the invention in a concentration equal to or greater than 60 pg/ml, preferably equal to or greater than 75 pg/ml, with values comprised, for example, between 50 and 100 pg/ml, preferably between 60 and 90 pg/ml .

In one embodiment, TGF-α can be present in the serum for use according to the invention in a concentration equal to or less than 50 pg/ml, preferably between 15 and 45 pg/ml, advantageously between 20 and 40 pg/ml.

In one embodiment, PDGF can be present in the serum for use according to the invention in a concentration equal to or greater than 7000 pg/ml, preferably equal to or greater than 9000 pg/ml, with values comprised, for example, between 5000 and 14000 pg/ml, preferably between 7000 and 10000 pg/ml.

In one embodiment, FGF can be present in the serum for use according to the invention in a concentration equal to or greater than 300 pg/ml, preferably equal to or greater than 400 pg/ml, with values comprised, for example, between 200 and 500 pg/ml.

In one embodiment, IL-10 can be present in the serum for use according to the invention in a concentration equal to or greater than 7 pg/ml, preferably equal to or greater than 9 pg/ml, with values comprised, for example, between 5 and 15 pg/ml, preferably between 7 and 13 pg/ml.

In one embodiment, IL-13 can be present in the serum for use according to the invention in a concentration equal to or greater than 130 pg/ml, preferably equal to or greater than 150 pg/ml, with values comprised, for example, between 130 and 300 pg/ml, preferably between 130 and 200 pg/ml.

In one embodiment, BDNF is present in the serum for use according to the invention in a concentration equal to or greater than 10000 pg/ml, preferably equal to or greater than 12000 pg/ml, advantageously equal to or greater than 13000 pg/ml.

In one embodiment, BDNF is comprised in the serum in a concentration comprised between 9500 and 20000 pg/ml, preferably between 10000 and 18000 pg/ml.

In one embodiment, GDNF is present in the serum for use according to the invention in a concentration equal to or greater than 1 pg/ml, preferably equal to or greater than 1.1 pg/ml.

In one embodiment, GDNF is present in the serum in a concentration comprised between 0.5 and 4 pg/ml, preferably between 1 and 3 pg/ml.

In one particularly preferred embodiment, the blood serum for use according to the present invention comprises:
- NGF-β, preferably in a concentration equal to or greater than 5 pg/ml;
- EGF, preferably in a concentration equal to or greater than 500 pg/ml;
- TGF-α, preferably in a concentration equal to or greater than 50 pg/ml;
- BDNF, preferably in a concentration equal to or greater than 9500 pg/ml; e
- GDNF, preferably in a concentration equal to or greater than 0.5 pg/ml.

In one embodiment, the serum of the invention comprises BDNF, preferably present in a concentration equal to or greater than 9500 pg/ml, optionally in combination with GDNF, preferably present in a concentration equal to or greater than 0.5 pg/ml.

In one embodiment, the serum comprising BDNF, preferably present in a concentration equal to or greater than 9500 pg/ml, also comprises NGF-β in a concentration equal to or less than 5 pg/ml, preferably comprised between 0.5 and 5 pg/ml, advantageously comprised between 0.7 and 4 pg/ml.

In one embodiment, the serum comprises BDNF, NGF-β, GDNF, EGF and TGF-α.

In one embodiment of the invention, the blood serum for use described here can be serum from umbilical cord blood. Hereinafter, for the sake of brevity, the term "cord" will be used to indicate the origin of a certain raw material from an umbilical cord; thus, for example, "cord blood" means blood of an umbilical cord, "cord serum" means serum originating from blood of an umbilical cord, etc. The rationale for preferring cord serum for the use according to the invention is given by the high content in growth factors that are naturally contained therein. Alternatively, the blood serum for use described here can also be serum from the blood of an adult organism.

In another embodiment of the invention, the blood serum for use as described here can preferably derive from human blood.

In another embodiment of the invention, the blood serum for use as described here can be obtainable by means of the method of preparation that will be described below.

The blood serum according to the present invention, containing growth factors and/or cytokines, has use in the treatment of neurodegenerative pathologies in the ophthalmologic field. Furthermore, the serum can be used in the treatment of ophthalmologic pathologies whose progression is linked to the activation of neuroinflammatory processes, for example retinitis pigmentosa, age-related macular degeneration (AMD), diabetic retinopathies or Usher syndrome.

In particular, the blood serum as described here can be used for the treatment of degenerative pathologies of the retina and degenerative pathologies of the optic nerve. Over time, these types of degenerative ophthalmologic diseases lead to a considerable reduction in the visual field and permanent damage to vision. The use of the serum of the invention to treat such pathologies is thus extremely advantageous, since by containing the neuronal damage and/or slowing down the progression thereof, it enables subjects affected by such pathologies to improve their quality of life.

In greater detail, the serum of the invention can be used for the treatment of glaucoma: in one embodiment, the serum of the invention can be used for the treatment of primary open-angle glaucoma; in another embodiment, the serum of the invention can be used for the treatment of primary closed-angle glaucoma.

Another aspect of the present invention relates to a method for preparing the blood serum for the use described herein. The method according to the invention comprises the steps of:
i. analysing the concentration of NGF-β in serum samples obtained from corresponding blood samples; and
ii. selecting the serum samples having a concentration of NGF-β equal to or greater than 5 pg/ml.

In step i. of the method of the invention, characterisation of the serum takes place: the serum samples are analysed to determine the concentration of NGF-β. Based on the concentration of NGF-β determined for the various samples, in step ii. of the method only the samples whose concentration of NGF-β is greater than 5 pg/ml are selected.

In one embodiment, in step i. the concentration of any other growth factors and/or cytokines of interest can optionally also be evaluated; in this case, the selection of the serum samples in step ii. can also be made on the basis of the concentration of these additional growth factors and/or cytokines (as well as of NGF-β), which can preferably be greater than the threshold concentrations specified above. In one embodiment, in step i. of characterising the serum, it is possible to determine the concentration of one or more molecules selected from the group consisting of: EGF, TGF-α, PDGF, FGF, IL-10 and IL-13, BDNF and GDNF.

The method for preparing the blood serum for use comprises the further steps of:
i. analysing the concentration of BDNF in serum samples obtained from corresponding blood samples; and
ii. selecting the serum samples having a concentration of BDNF equal to or greater than 9500 pg/ml.

In step i. of the method of the invention, characterisation of the serum takes place: the serum samples are analysed to determine the concentration of BDNF. Based on the concentration of BDNF determined for the various samples, in step ii. of the method only the samples whose concentration of BDNF is greater than 9500 pg/ml are selected.

The serum samples subjected to analysis in step i. of the method can be obtained from blood according to standard procedures, known and commonly used in the art. The blood samples are typically allowed to coagulate at room temperature for a period of about 2-3 hours (so that the solid component of the blood sediments), then subjected to centrifugation, generally at a speed comprised between 2500 rpm and 3500 rpm. The serum (supernatant) is then collected, for example by aspiration.

In one embodiment of the method, the serum samples selected during step ii. can be frozen for a period of no longer than 24 months. The serum samples can preferably be frozen at a temperature comprised between -20°C and -85°C.

As is known to the person skilled in the art, the storage temperature of the serum samples is directly correlated to the maximum period of storage thereof. Therefore, for example, the serum samples can be kept at -20°C for a maximum period of 6 months, or at -80°C for a maximum period of 48 months.

The samples can preferably be kept frozen (preferably at around -70°C) for about 6-8 weeks.

Maintaining a frozen situation for a period of at least 6-8 weeks is referred to as the "quarantine period" and makes it possible to verify that the collected serum has no contaminations and is maintained sterile.

In order to proceed to use the serum of the invention, one or more selected serum samples may optionally be joined together (after thawing, if they were frozen) and used as such or for the production of ophthalmic preparations for the treatment of neurodegenerative pathologies or ophthalmologic pathologies whose progression is linked to the activation of neuroinflammatory processes.

The blood samples from which the serum used in the method of the invention is obtained can preferably be blood samples from a human umbilical cord.

The method according to the present invention enables the preparation of a blood serum that is effective in the neuroprotection of diseased ocular tissue and as an anti-inflammatory agent, thanks to a concentration of growth factors and/or cytokines exceeding the values specified above as the minimum threshold.

The serum for use in the treatment of neurodegenerative ophthalmologic pathologies according to the invention can naturally contain the growth factors and cytokines specified herein. The natural origin of said growth factors and cytokines appears to be correlated to a bioavailability of the same exceeding that of the corresponding products obtained synthetically (for example by means of recombinant synthesis technologies, not yet commercially available, but available for study).

The serum according to the present invention can be used as such, i.e. as an active principle in pure form, for the uses described herein, or else it can be included within a composition.

Therefore, the present invention relates to a composition comprising the serum for use in the treatment of neurodegenerative ophthalmologic pathologies or for the treatment of ophthalmologic pathologies whose progression is linked to the activation of neuroinflammatory processes as described herein and, optionally, one or more additional substances. As is known in the art, such substances can typically be selected from the group consisting of excipients, carriers and preservatives acceptable for human use. The composition can preferably comprise the serum as described herein and a physiological aqueous solution, typically having a pH comprised between 7 and 8. The composition can be in liquid form, in gel form, in hydrocolloid form, in spray form, for example a nasal spray; the composition can preferably be in liquid form. Furthermore, the composition is typically used topically, by administration inside the conjunctival fornix or transnasally through the nasal mucosa.

The composition can be prepared in a form that is appropriate for use in ophthalmology: for example, the composition can be in the form of a collyrium, eye or nasal drops, eye or nasal ointment, eye or nasal gel or nasal spray; the composition can preferably be in the form of a collyrium. If the serum is formulated in liquid form, for example through the use of a physiological aqueous solution, the concentration of the growth factors and cytokines described above will be diluted by a factor comprised between 1:2 and 1:10, for example a dilution factor of 1:5.

Therefore, in a preferred embodiment, wherein the serum formulated in liquid form is diluted by a factor of 1:2, some concentration values for some of the factors specified above, calculated on the basis of a 1:2 dilution are as set forth here below:
- EGF in a concentration equal to or greater than 250 pg/ml, or equal to or greater than 350 pg/ml, or equal to or greater than 500 pg/ml;
- PDGF in a concentration equal to or greater than 2500 pg/ml, or equal to or greater than 3500 pg/ml, or equal to or greater than 4500 pg/ml;
- FGF in a concentration equal to or greater than 100 pg/ml, or equal to or greater than 150 pg/ml, or equal to or greater than 200 pg/ml;
- IL-10 in a concentration equal to or greater than 2.5 pg/ml, or equal to or greater than 3.5, or equal to or greater than 4.5 pg/ml;
- IL-13 in a concentration equal to or greater than 50 pg/ml, or equal to or greater than 65 pg/ml, or equal to or greater than 75 pg/ml;
   - BDNF in a concentration equal to or greater than 4750 pg/ml, or equal to or greater than 5000 pg/ml, preferably equal to or greater than 6000 pg/ml,
   - GDNF in a concentration equal to or greater than 0.25 pg/ml, or equal to or greater than 0.5 pg/ml, or equal to or greater than 0.55 pg/ml.

In another embodiment, wherein the serum formulated in liquid form is diluted by a factor of 1:10, some concentration examples for some of the factors specified above, calculated on the basis of a 1:2 dilution, are as set forth here below:
- EGF in a concentration equal to or greater than 50 pg/ml, or equal to or greater than 70 pg/ml, or equal to or greater than 100 pg/ml;
- PDGF in a concentration equal to or greater than 500 pg/ml, or equal to or greater than 700 pg/ml, or equal to or greater than 900 pg/ml;
- FGF in a concentration equal to or greater than 20 pg/ml, or equal to or greater than 30 pg/ml, or equal to or greater than 40 pg/ml;
- IL-10 in a concentration equal to or greater than 0.5 pg/ml, or equal to or greater than 0.7, or equal to or greater than 0.9 pg/ml;
- IL-13 in a concentration equal to or greater than 10 pg/ml, or equal to or greater than 13 pg/ml, or equal to or greater than 15 pg/ml
   - BDNF in a concentration equal to or greater than 950 pg/ml, or equal to or greater than 1000 pg/ml, or equal to or greater than 1200 pg/ml.
   - GDNF in a concentration equal to or greater than 0.05 pg/ml, or equal to or greater than 0.1 pg/ml, preferably equal to or greater than 0.11 pg/ml.

In the embodiment wherein the serum formulated in liquid form is diluted by a factor of 1:5 or any other factor comprised between 1:2 and 1:10, or by other factors not specified above, the concentrations of the growth factors and cytokines present in the composition can be easily calculated, as previously described, by a person having ordinary skill in the art.

The invention also relates to a method for treating a human or animal subject affected by a neurodegenerative ophthalmologic pathology, in particular degenerative pathologies of the retina or of the optic nerve, for example glaucoma, or affected by one or more ophthalmologic pathologies whose progression is linked to the activation of neuroinflammatory processes, which comprises a step of administering a therapeutically effective dose of the serum of the invention to the subject in need thereof.

Administration is preferably topical, by administration inside the conjunctival fornix or transnasally through the nasal mucosa.

The serum can be administered as such or formulated as a composition.

In one embodiment, the method comprises the administration, to said subject, of a therapeutically effective dose of a composition comprising the serum of the invention. Said composition can be in liquid form, in gel form, in hydrocolloid form or in spray form. The composition is preferably a collyrium, eye or nasal drops, eye or nasal ointment, eye or nasal gel.

Furthermore, the composition is typically used topically, by administration inside the conjunctival fornix or transnasally through the nasal mucosa.

The composition to be administered preferably comprises the serum of the invention and a physiological aqueous solution, typically having a pH comprised between 7 and 8. The therapeutically effective dose and/or dosage of administration of the composition in liquid form comprising the serum of the invention, depends on the concentration of the growth factors and cytokines present within it. Said dose and/or dosage can be easily deduced by a person skilled in the art.

Some examples relating to the preparation of the cord blood serum and a preparation containing it (collyrium) are provided below, along with an in vitro assessment on a cellular model and an in vivo assessment on an animal model of the effectiveness of said serum or collyrium in the treatment of neurodegenerative processes. Further examples relate to the use of said serum or collyrium for treating patients affected by ophthalmologic neurodegeneration. The examples are obviously not to be construed as limiting the scope of protection of patent application.

### EXAMPLES

### Example 1.0: Preparation of cord blood serum

Samples of umbilical cord blood were donated by some women immediately after giving birth, subject to acceptance of an informed consent and authorisation of the use of the blood itself for transplantology purposes, for clinical or research use and/or intellectual protection.

The cord blood was taken from placenta vessels at the time of parturition, collected in dedicated test tubes clinically validated for that use and sent to the regional cord blood bank of the S. Orsola - Malpighi University Hospital and Polyclinic.

The cord blood samples were first allowed to coagulate for 2 hours at room temperature and were then centrifuged at 3000 rpm in a refrigerated centrifuge at +4/+10°C for 15 minutes. The serum samples derived from the cord blood samples (i.e. the supernatant obtained at the end of centrifugation) were collected by aspiration, placed in test tubes and analysed in order to evaluate the concentration of NGF-β and other growth factors and cytokines.

The selected serum samples, having an NGF-β concentration of greater than 5pg/ml, were placed in storage tubes and kept at -80°C until the time of their use in the preparation of the collyrium.

The concentration of a specific growth factor (EGF) was evaluated at the end of the storage period and beyond, and demonstrated to be substantially constant also after six months of maintenance at -80°C.

### Example 1.1: Preparation of a cord blood serum-based collyrium

The cord serum samples stored at -80°C were thawed and, working in a laminar flow hood in a class D controlled environment, more cord serum samples (same blood group and clinically validated) were assembled in such a way as to obtain four "pools", each containing a volume of serum of no less than 30 ml.

The concentration in growth factors and interleukins in the four pools produced was the following (values expressed in pg/ml).

| | **Pool 1** | **Pool 2** | **Pool 3** | **Pool 4** |
|---|---|---|---|---|
| NGF-β | 10 | 8.5 | 7.0 | 6.0 |
| EGF | 763.5 | 744 | 1200 | 1200 |
| TGF-α | 78.5 | 64.0 | 96.5 | 99.0 |
| PDGF-BB | 9900 | 8700 | 9600 | 12200 |
| FGF-basic | 521.0 | 384.5 | 482.0 | 221.5 |
| IL-10 | 9.0 | 7.5 | 14.5 | 6.0 |
| IL-13 | 207.0 | 125.0 | 312.0 | 139.5 |

In the laminar flow hood, each pool was diluted with a sterile physiological solution at pH 7.4, whose volume was equal to 4 times the volume of the pool (1 to 5 dilution), filtered and dispensed in 1 ml single-dose vials.

The individual vials were labelled and every lot (made up of 15 vials marked with sequential numbers) was packaged in a transparent hermetically sealed plastic bag, to which an identification label was affixed. A search for aerobic and anaerobic bacteria and fungi was carried out on every lot using BacT/ALERT^{®} bottles (Biomerieux) validated for the blood component concerned. The lots of collyrium were made available for experimental trials on patients only after the negative microbiological results were verified.

### Example 1.2: In vivo evaluation on patients

### Computerised examination of the visual field (VF)

The computerised examination of the visual field (VF) measures the vision of the space that surrounds the eye and serves to evaluate the presence of alterations in visual field sensitivity attributable to retinal and/or optic nerve pathologies. The alterations in visual field sensitivity are examined by evaluating perception in various points of the normal visual field.

The VF examination is performed by means of a localised light stimulation of the retina: practically, an assessment is made of the patient's ability to perceive and respond to light stimuli of varying form and intensity projected onto or activated on a half-dome shaped screen placed in front of the patient's eyes at a distance of 33 cm. The examination is performed on each eye individually (thus occluding the patient's eye that is not undergoing examination), lasts about 15 minutes per eye and is painless. The instrument used for the analysis consists of a dome-shaped screen with a white background onto which light signals are projected: when the patient perceives the light signal, he or she indicates this to the operator, for example by pressing a luminous or acoustic pushbutton. In the present study, a 2003 Carl Zeiss Meditec Humphrey HFA II 745-2205 instrument was used.

The data collected during the examination are processed by means of software that extrapolates the perimeter indexes of interest. In the present study use was made of the instrument software of the 2003 Carl Zeiss Meditec Humphrey HFA II 745-2205, upgrades 12.5/12.6.

The perimeter indexes MD (mean deviation) and PSD (pattern standard deviation) were considered in the present study. The MD value is the mean difference between the retinal sensitivity of the subject undergoing examination and what is considered normal sensitivity, corrected for age, and calculated as the arithmetic mean for all points of the visual field tested during the examination. MD is measured in decibels (dB) and is considered normal for values comprised between -2 and +2 dB: the higher the (positive) MD value, the greater the visual impairment. The PSD value is the variance of the mean deviation, i.e. it indicates how the mean deviation is distributed in the visual field. PSD is indicative of the presence of a localised visual defect and thus expresses the inhomogeneity of the defect. PSD is also measured in decibels (dB): the higher the value, the greater the distribution of the defect in the quadrants of the visual field.

In the studies described below, an assessment was made of the pattern in the MD and PSD values of visual field sensitivity in patients examined in recent years, before and after treatment with the serum of the invention.

### Patients

The clinical study was conducted on two patients undergoing treatment at the Operational Ophthalmology Unit of the *Alma Mater Studiorum* University of Bologna and the S. Orsola - Malpighi University Hospital of Bologna.

Patient 1, a 66-year-old male, was affected by diabetes mellitus type (I), had been undergoing pharmacological therapy since 2006, and presented with diabetic retinopathy and primary open-angle glaucoma (POAG), diagnosed in January 2014 and treated with topical intraocular-pressure reducing drugs, effective in maintaining the value of intraocular pressure within normal limits. The symptoms the patient complained of were pain (described as irritation, burning, feeling of sand in the eyes, discomfort) in both eyes. The biomicroscopic examination did not reveal any concomitant keratopathy; observation at the slit-lamp showed no corneal epithelial defects.

Patient 2, a 60-year-old female, had been affected by primary open-angle glaucoma (POAG) for 10 years, regularly underwent tonometry and was undergoing therapy with topical intraocular-pressure reducing drugs. The symptoms the patient complained of were pain (described as irritation, burning, feeling of sand in the eyes, discomfort), intense in the right eye and bearable in the left eye; the therapies planned in the six months prior to observation (tear substitutes, anti-inflammatories, topical cortisone) had produced only a minimal effect on the symptoms. The biomicroscopic examination did not reveal any concomitant keratopathy; observation at the slit-lamp showed no corneal epithelial defects.

### Treatment and results

Patient 1 began treatment in June 2016 and was treated for 2 months with 2 lots of cord blood serum-based collyrium, prepared from serum pools 1 and 2; each lot included 30 vials, for a total of 60 administrations. Every vial contained an amount of collyrium necessary for one day of treatment, for both eyes: the entire content was administered within 12 hours after the vial had been opened and the vial was stored at a temperature comprised from 4°C to 10°C between one administration and another. The collyrium was administered 8 times a day, approximately every 2 hours, from the time of awakening to before bedtime, by instilling 1 drop in each eye at every administration.

In September 2016, patient 1, when undergoing a check-up, reported a significant attenuation in the symptoms of irritation and pain, which were more bearable and on some days not even perceptible.

With reference to the accompanying figure 1, which shows the trend in the MD values of visual field sensitivity measured in examinations carried out in January 2014, June 2016 and September 2016, a progressive worsening in the MD values can be observed in the period between 2014 and June 2016, followed by an improvement in the period of June-September 2016. This period coincides with the treatment with the cord blood serum-based collyrium (the start of the treatment is indicated in the graph with an arrow corresponding to June 2016).

Similarly, with reference to the accompanying figure 2, which shows the trend in the PSD values of visual field sensitivity measured in examinations carried out in January 2014, June 2016 and September 2016, a progressive worsening (increase) in the PSD values can be observed in the period between 2014 and June 2016. In the period June-September 2016, in which the patient was treated with the collyrium based on cord blood serum (the start of the treatment is indicated in the graph with an arrow corresponding to June 2016), by contrast, an improvement (decrease) in the PSD values was detected for the left eye, whereas the PSD values for the right eye remained substantially unchanged, indicating in any case a positive effect of the treatment with the serum of the invention.

Patient 2 started the treatment in March 2016 and was treated for 2 months with 2 lots of cord blood serum-based collyrium, prepared from serum pools 3 and 4; each lot included 15 vials, for a total of 60 administrations. Every vial contained the amount of collyrium necessary for two days of treatment, for only one eye (right eye): the entire content was administered within 36 hours after the vial had been opened and the vial was stored at a temperature comprised from 4°C to 10°C between one administration and another. The collyrium was administered 8 times a day, approximately every 2 hours, from the time of awakening to before bedtime, by instilling 1 drop at every administration, in the eye to be treated.

In June 2016, patient 2, when undergoing a check-up, reported a significant attenuation in the symptoms of irritation and pain, which were bearable to the point that she could engage in her everyday activities in an acceptable manner.

With reference to the accompanying figure 3, which shows the trend in the MD values of visual field sensitivity measured in examinations carried out in December 2006, June 2008, February 2011, September 2012, November 2013, March 2016, June 2016 and September 2016, a progressive worsening of the MD values can be observed in the period between 2006 and March 2016, followed by an improvement in said values in the period March-June 2016. This period coincides with the treatment with the cord blood serum-based collyrium (the start of the treatment is indicated in the graph with an arrow corresponding to March 2016). The MD values recorded in September 2016, 4 months after the end of the treatment, were still significantly improved and close to the MD values recorded in the first years of the disease.

Similarly, with reference to the accompanying figure 4, which shows the trend in the PSD values of visual field sensitivity measured in examinations performed in December 2006, June 2008, February 2011, September 2012, November 2013, March 2016, June 2016 and September 2016, a progressive worsening (increase) of the PSD values can be observed in the period between 2006 and March 2016. In the period March-June 2016, in which the patient was treated with the cord blood serum-based collyrium (the start of the treatment is indicated in the graph with an arrow corresponding to March 2016), by contrast, an improvement (decrease) in the PSD values was found. The PSD values recorded in September 2016, 4 months after the end of the treatment, were still significantly improved and even lower than the PSD values recorded in the first years of the pathology (2006).

A further interesting observation results from a comparison between the MD and PSD values for the right eye (treated with the cord blood serum-based collyrium) and left eye (not treated). Not only did the eye undergoing treatment with the serum-based collyrium (right, OD in the figure) show a significant improvement in the MD and PSD values, but the untreated contralateral eye (left, OS in the figure) also showed an improvement in the MD and PSD values. This result suggests the existence of a neural cross-talk relationship between the two eyes.

### Example 2.0: Preparation of cord blood serum

The umbilical cord blood samples were donated by a group of several women, different from the ones of example 1.0, immediately after giving birth, subject to acceptance of an informed consent and authorisation of the use of the blood itself for transplantology purposes, for clinical or research use and/or intellectual protection.

The cord blood was taken from placenta vessels at the time of parturition, collected in dedicated test tubes clinically validated for that use and sent to the regional cord blood bank of the S. Orsola - Malpighi University Hospital and Polyclinic.

The cord blood samples were first allowed to coagulate for 2 hours at room temperature and were then centrifuged at 3000 rpm in a refrigerated centrifuge at +4/+10°C for 15 minutes. The serum samples derived from the cord blood samples (i.e. the supernatant obtained at the end of centrifugation) were collected by aspiration, placed in test tubes and analysed in order to evaluate the concentration of NGF-β, EGF and other growth factors and cytokines.

The selected serum samples, having an EGF concentration greater than 500pg/ml, were placed in storage tubes and kept at -80°C until the time of their use in the preparation of the collyrium.

The concentration of a specific growth factor (EGF) was evaluated at the end of the storage period and beyond, and demonstrated to be substantially constant also after six months of maintenance at -80°C. The cord serum samples stored at -80°C were thawed and, working in a laminar flow hood in a class D controlled environment, more cord serum samples (same blood group and clinically validated) were assembled in such a way as to obtain a "pool" containing a volume of serum of no less than 30 ml.

The concentration in growth factors and interleukins in the pool produced was the following (values expressed in pg/ml).

| | **Pool 1** |
|---|---|
| NGF-β | 0.70 |
| EGF | 890 |
| TGF-α | 66.6 |
| TFG-β1 | 64271 |
| TGF- β2 | 1056 |
| TGF- β3 | 552 |
| PDGF-BB | 7103 |
| FGF-basic | 77.5 |
| IL1- β | 33957 |
| IL-4 | 0.38 |
| IL-6 | 116785 |
| IL-10 | 15.4 |
| IL-13 | 262.7 |
| IGF1 | 13.57 |
| IGF2 | 68.54 |
| VEGF | 635 |

### Example 2.1: In vivo evaluation in an animal model with induced retinal degeneration

The effectiveness of pure cord blood serum, obtained as in example 2.0, according to the present invention, was tested in the treatment of neurodegenerative processes in an animal model with induced retinal degeneration.

The use of high intensity light on albino rats induces the death of photoreceptors, which consequently leads to the progression of neurodegenerative processes.

Light damage (LD) on the retina is a well-established model for testing the effectiveness of neuroprotective agents.

In particular, the model used shows a more sensitive dorsal area called "hot spot": this area is the zone where degeneration begins. Degeneration is subsequently guided by different diffusion factors that lead to neuroinflammation, which extends with the passing of time. Visual functionality is also strongly compromised: for this reason the effectiveness of any neuroprotective agent can be assessed by means of such a method, through both electrophysiological and morphological methods.

The experiment was conducted on a total of 24 albino rats *(Sprague Dawley* strain), all of an age comprised between 3 and 4 months. The animals were born and reared in the Applicant's own facility, under low luminance conditions (5 lux) and under conditions of alternating light and darkness (12 hours of light alternating with 12 hours of darkness); the animals analysed were divided into four groups:
1) normal control group not treated with the serum of the present invention ;
2) normal control group treated with the serum of the present invention ;
3) control group with retinal degeneration induced by exposure to light;
4) group with retinal degeneration induced by exposure to light with one eye treated with the serum of the present invention and one untreated eye.

The treatment with the serum of the present invention in the groups of rats indicated began seven days before exposure to high intensity light. In the case of the rats in group 4, only one eye was treated with a single drop (about 10 µL) three times a day, leaving the other eye untreated as a control. Following exposure to high intensity light, the rats were monitored and treated with the same protocol for the duration of seven days.

### Light damage

The albino rats of groups 3 and 4 were placed one by one in a cage with lights positioned in both the upper and lower parts in such a way as to ensure an environment under isoluminance (1000 lux) inside the cage. The litter was removed to prevent the rats from hiding their eyes from the light. The exposure to light began at the start of the natural daytime phase, that is, immediately after 12 hours of darkness. The animals were exposed to 1000 lux of light for 24 consecutive hours, after which they were immediately positioned back in their normal cages, with normal conditions being reestablished.

### Electrophysiological analysis

Subsequently, one week after the exposure to damaging light, electroretinograms (fERGs) were recorded in response to flashes of increasing luminance in order to assess the visual functionality of the retina. Prior to recording of the electroretinogram, the albino rats were kept in darkness for 12 hours and the analyses were performed in a darkened chamber.

The rats were anaesthetised by intraperitoneal injection of Ketamine/Xylazine (10- 1.2 mg/100g) and mounted inside a stereotaxic apparatus positioned within the opening of a Ganzfeld dome (Biomedica Mangoni, Pisa, Italy). The body temperature was maintained at 37.5 °C using a heating plate controlled by a rectal probe.

The corneas were anaesthetised with a drop of novocaine and the pupils were dilated with 1% tropicamide.

The flash generating unit used produces flashes with an intensity comprised between 0.001 and 100 cd/m². The responses were recorded over 300 ms plus 25 ms of pre-test baseline and amplified in a differential manner, filtered with a band-pass filter at 0.3 - 300 Hz and, finally, digitised at intervals of 0.25 to 0.3 ms *via* a PC interface (LabVIEW 8.2, National Instruments, Milan, Italy).

A-waves, b-waves and oscillatory potentials were analysed.

The amplitudes of the a-wave and of the b-wave, recorded at two flash intensities, are shown in Figure 5. In figure 5, the letters A and B indicate the a and b waves recorded for healthy eyes with and without treatment with the collyrium: both the treated eyes and the untreated ones show comparable amplitude responses. The letters C and D, by contrast, indicate the a and b waves recorded for eyes subjected to damaging light which were treated with the collyrium and not treated with the collyrium and the control eyes. In accordance with the expected results, the amplitude of both the a-wave and b-wave were greatly reduced as a result of the light-induced damage. However, the treatment with the collyrium enabled the functionality of the retina to be largely preserved in the treated eye compared to the untreated counterpart or control sample.

As far as the oscillatory potentials are concerned, no significant effect was detected.

At the end of the recording session, the animals were sacrificed. The eyes and retinas were removed and used for the subsequent morphological analysis.

### Morphological analysis

For the preparation of the retina, the eyes were sectioned and subsequently fixed by immersion in a fixative buffer of 4% paraformaldehyde at a temperature of 4 °C for 24 hours.

After three washes with a phosphate-buffered saline (PBS) solution, the eyes were left overnight in a 15% sucrose solution to assure the future cryoprotection thereof. The eyes were then inserted into a mounting medium (Tissue Tek OCT compound; Sakura Finetek, Torrance, CA) by rapid freezing in liquid nitrogen. Subsequently, 20mm cryosections were obtained, extending from the dorsal to the ventral region.

The sections were then mounted on gelatine and slides coated with poly-L-lysine and subsequently dried overnight in an oven at 50 °C and stored at -20 °C.

For the purpose of evaluating the cell layers, the sections were marked with the DNAspecific dye bisbenzimide (Calbiochem, La Jolla, CA) and incubated for 2 minutes in a 1:10000 solution in 0.1 M PBS. Sections cut in such a way as to pass through the papilla (origin of the optic nerve) were chosen. Every section was scanned from the dorsal to the ventral edge of the retina.

In retinas damaged by intense light, the photoreceptors initially die in the area of the "hot spot" and neuronal death progressively increases in the adjacent areas and as a result the thickness of the outer nuclear layer (ONL) is markedly reduced.

Photoreceptor survival was then estimated by measuring the thickness of the outer nuclear layer (ONL). In particular, the ONL thickness was recorded over the entire extent of the retina, from the dorsal to the ventral region passing through the papilla; said thickness was measured at intervals of 0.40 mm. The ratio between the ONL thickness and that of the retina was used as a measure of the thickness of the ONL itself, rather than using the absolute thickness of the ONL (mm), in order to compensate for any oblique sections. Furthermore, the extent of the "hot area" was measured in order to assess the degree of damage and the effectiveness of the protection. All measurements were performed using Image J 2.0 software.

As may be observed in Figure 6A, in treated eyes, in the dorsal retina the ONL is better preserved than in the corresponding untreated eyes, whereas the ventral retina appears similar. Furthermore, the extent of the "hot spot" was measured from the dorsal periphery to the optic disc and the ratio of the two values is shown in Figure 6B. The extent of the damaged zone is significantly reduced in the treated eyes.

The results of this study thus confirm the hypothesis that the collyrium based on umbilical cord blood can be successfully used to mitigate retinal neurodegenerative processes. Functionality is partially maintained and the morphology of the retina remains in good conditions compared to untreated animals exposed to damage.

### Example 3.0: Preparation of cord blood serum

Samples of umbilical cord blood were donated by a group of several women, different from the ones of examples 1.0 and 2.0, immediately after giving birth, subject to acceptance of an informed consent and authorisation of the use of the blood itself for transplantology purposes, for clinical or research use and/or intellectual protection.

The cord blood was taken from placenta vessels at the time of parturition, collected in dedicated test tubes clinically validated for that use and sent to the regional cord blood bank of the S. Orsola - Malpighi University Hospital and Polyclinic.

The cord blood samples were first allowed to coagulate for 2 hours at room temperature and were then centrifuged at 3000 rpm in a refrigerated centrifuge at +4/+10°C for 15 minutes. The serum samples derived from the cord blood samples (i.e. the supernatant obtained at the end of centrifugation) were collected by aspiration, placed in test tubes and analysed in order to evaluate the concentration of NGF-β and other growth factors and cytokines.

The selected serum samples, having a BDNF concentration greater than 9500 pg/ml, were placed in storage tubes and kept at -80°C until the time of their use in the preparation of the collyrium.

The concentration of a specific growth factor (EGF) was evaluated at the end of the storage period and beyond, and demonstrated to be substantially constant also after six months of maintenance at -80°C.

The cord serum samples stored at -80°C were thawed and, working in a laminar flow hood in a class D controlled environment, more cord serum samples (same blood group and clinically validated) were assembled in such a way as to obtain a "pool" containing a volume of serum of no less than 30 ml.

The concentration in growth factors and interleukins in the pool produced was the following (values expressed in pg/ml).

| | **Pool 1** |
|---|---|
| NGF-β | 2.65 |
| BDNF | 13618.95 |
| GDNF | 1.34 |
| EGF | 744.06 |
| TGF-α | 39.1 |

### Example 3.1: Evaluation of an in vitro cellular model

It is by now a commonly acknowledged fact that glial cells play a role, vis-à-vis neurons, in protecting against various neurological insults.

In the event of insults to the retina, Müller cells, which are the predominant glial element in the retina, undergo significant morphological, cellular and molecular changes, thus protecting the retina against further damage. Müller cells express and are sensitive to the action of growth factors, are neurotransmitter transporters and produce antioxidant agents that could have an important role in preventing cytotoxic damage to the neurons of the retina. Furthermore, Müller cells come into contact with endothelial cells to facilitate the process of neovascularisation during hypoxic conditions.

The experiments were conducted with the rMC-1 cell line (retinal glial cells, rat Müller cells).

This cell line expresses GFAP (glial fibrillary acidic protein) and CRALBP (cellular retinaldehyde-binding protein), markers that are present in Müller cells, and are immortalised by transfection with a viral oncogene SV-40 T.

### Cell culture: rMC-1 cell model

The rMC-1 cells were purchased from ABM, Materials for Life (Richmond, BC, Canada) and cultured in 24-well plates, as recommended by the supplier, using a specific culture medium recommended by the supplier.

### Induction of oxidative damage and cell viability test

Preliminary experiments led to the choice of using scalar concentrations of H₂O₂ for cultures under a condition of semi-confluence (80% of the well covered). Briefly, the cultures were subjected to the induction of oxidative damage by adding 10, 50, 100, 200, 500 µM to the culture medium for a contact time of 3, 6 and 24 hours, in a CO₂ incubator at 37°C.

An H₂O₂ concentration of 100 and 200 µM was chosen to be used in further experiments, since a lower concentration of H₂O₂ had negligible effects on cell viability and greater concentrations of H₂O₂ make the results difficult to reproduce, as they induce a significant cell detachment. Similar considerations oriented the choice of 6 hours of incubation for the damage by H₂O₂.

One line of experiments was conducted using 10% Fetal Bovine Serum (FBS) in the culture medium, as the standard composition for maintaining the rMC-1 cell culture, whilst a line of experiments was conducted in parallel using 10% cord blood serum (lot dosed as described in example 3.0) in the culture medium, as a comparative percentage.

The viability test was performed using the MTT test (3-(4,5-dimethylthiazol-2-yl)-2,5-diphenyltetrazolium bromide), a method that is based on the intracellular reduction of tetrazolium salts by the mitochondrial enzyme succinate dehydrogenase (SDH) into crystals of a dark blue product called formazan. The reaction can thus occur only in metabolically active cells and the value of optical density (O.D.), obtained by means of a spectrophotometric reading, can be correlated to the amount of viable cells present.

### Digital images and statistical analysis

Phase contrast images of the central parts of at least one well replicated by each experimental treatment were recorded to show the cell morphology; they were recorded as .tif files using an Axiovert 25 photomicroscope with inverted CFL technology (Carl Zeiss Microscopy LLC, Thornwood, NY), equipped with a Coolpix digital camera. The results of the cell viability analysis are shown in Figure 7 and are expressed as the mean ± standard deviation (SD). The statistical analyses were performed using GraphPad Prism 6 software. The optical density values were normalised with the RMC-1 control cultured in a medium without FBS and CBS. The statistical analysis was performed by means of one-way ANOVA test and *Tukey's* multiple comparison test. The results demonstrate that H₂O₂ significantly reduces cell viability (at both concentrations); furthermore, the RMC-1s cultured in a medium with 10% CBS and treated with 200 µM H₂O₂ showed greater survival than RMC-1s cultured in the presence of FBS and treated with 200 µM H₂O₂.

### Example 4.0: Preparation of cord blood serum

The umbilical cord blood samples were donated by a group of several women, different from the ones of example 1.0, 2.0 or 3.0, immediately after giving birth, subject to the acceptance of an informed consent and authorisation of the use of the blood itself for transplantology purposes, for clinical or research use and/or intellectual protection.

The cord blood was taken from placenta vessels at the time of parturition, collected in dedicated test tubes clinically validated for that use and sent to the regional cord blood bank of the S. Orsola - Malpighi University Hospital and Polyclinic.

The cord blood samples were first allowed to coagulate for 2 hours at room temperature and were then centrifuged at 3000 rpm in a refrigerated centrifuge at +4/+10°C for 15 minutes. The serum samples derived from the cord blood samples (i.e. the supernatant obtained at the end of centrifugation) were collected by aspiration, placed in test tubes and analysed in order to evaluate the concentration of BDNF and other growth factors and cytokines.

The selected serum samples, having a BDNF concentration greater than 9500 pg/ml, were placed in storage tubes and kept at -80°C until the time of their use in the preparation of the collyrium.

The concentration of a specific growth factor (EGF) was evaluated at the end of the storage period and beyond, and demonstrated to be substantially constant also after six months of maintenance at -80°C.

### Example 4.1: Preparation of a cord blood serum-based collyrium

The cord serum samples stored at -80°C were thawed and, working in a laminar flow hood in a class D controlled environment, more cord serum samples (same blood group and clinically validated) were assembled in such a way as to obtain four "pools", a pool for every patient, each pool containing a volume of serum of no less than 30 ml.

The concentration in growth factors and interleukins in the four pools produced for the four patients was the following (values expressed in pg/ml).

| | **Patient 1** | **Patient 2** | **Patient 3** | **Patient 4** |
|---|---|---|---|---|
| | **(Pz1)** | **(Pz2)** | **(Pz3)** | **(Pz4)** |
| BDNF | 17333.79 | 12544.65 | 9993.79 | 19961.50 |
| NGF-β | 2.57 | 2.79 | 2.93 | 3.65 |
| GDNF | 1.17 | 1.83 | 1.71 | 1.49 |
| EGF | 917.92 | 544.89 | 655.79 | 894.79 |
| TGF-α | 22.52 | 22.90 | 32.98 | 33.55 |

In the laminar flow hood each pool was diluted with a sterile physiological solution at pH 7.4, whose volume was 4 times the volume of the pool (1 to 5 dilution), filtered and dispensed into 1 ml single-dose vials.

The individual vials were labelled and every lot (made up of 15 vials marked with sequential numbers) was packaged in a transparent hermetically sealed plastic bag, to which an identification label was affixed. A search for aerobic and anaerobic bacteria and fungi was carried out on every lot using BacT/ALERT^{®} bottles (Biomerieux) validated for the blood component concerned. The lots of collyrium were made available for experimental trials on patients only after the negative microbiological results were verified.

### Example 4.2: In vivo evaluation on patients

### Computerised examination of the visual field (VF)

The computerised examination of the visual field (VF) measures the vision of the space that surrounds the eye and serves to evaluate the presence of alterations in visual field sensitivity attributable to retinal and/or optic nerve pathologies. The alterations in visual field sensitivity are examined by evaluating perception in various points of the visual field.

The VF examination is performed by means of a localised light stimulation of the retina: practically, an assessment is made of the patient's ability to perceive and respond to light stimuli of varying form and intensity projected onto or activated on a half-dome shaped screen placed in front of the patient's eyes at a distance of 33 cm. The examination is performed on each eye individually (thus occluding the patient's eye that is not undergoing examination), lasts about 15 minutes per eye and is painless. The instrument used for the analysis consists of a dome-shaped screen with a white background onto which light signals are projected: when the patient perceives the light signal, he or she indicates this to the operator, for example by pressing a luminous or acoustic pushbutton. In the present study, a 2003 Carl Zeiss Meditec Humphrey HFA II 745-2205 instrument was used.

The data collected during the examination are processed by means of software that extrapolates the perimeter indexes of interest. In the present study use was made of the instrument software of the 2003 Carl Zeiss Meditec Humphrey HFA II 745-2205, upgrades 12.5/12.6.

The perimeter indexes MD (mean deviation) and PSD (pattern standard deviation) were considered in the present study. The MD value is the mean difference between the retinal sensitivity of the subject undergoing examination and what is considered normal sensitivity, corrected for age, and calculated as the arithmetic mean for all points of the visual field tested during the examination. MD is measured in decibels (dB) and is considered normal for values comprised between -2 and +2 dB; the higher the (positive) MD value, the greater the visual impairment. The PSD value is the variance of the mean deviation, i.e. it indicates how the mean deviation is distributed in the visual field. PSD is indicative of the presence of a localised visual defect and thus expresses the inhomogeneity of the defect. PSD is also measured in decibels (dB): the higher the value, the greater the distribution of the defect in the quadrants of the visual field.

In the studies described below, an assessment was made of the pattern in the MD and PSD values of visual field sensitivity in patients examined in recent years, before and after the treatment with the serum of the invention.

### Examination of intraocular pressure

Under normal conditions, the values of intraocular pressure (IOP) range from 12 to 20 mmHg, and depend essentially on the balance between the amount of aqueous humour produced in a continuous manner by the *pars plicata* of the ciliary body and the amount drained through the trabecular meshwork at the level of the anterior chamber angle (camerular angle). POAG, also known as chronic simple glaucoma, is characterised by: onset in adult-advanced age, IOP > 21 mmHg, anterior chamber angle open or normal in appearance, papillary excavation and reduction of the visual field. It is a chronic disease that progresses slowly, is generally bilateral with an insidious onset, and affects both sexes to an equal degree.

### Electroretinogram

A pattern electroretinogram (PERG) is a retinal biopotential evoked by a checkerboard or grating patterned stimulus; it mainly studies the functionality of ganglion cells and is obtained at low temporal frequencies (maximum 4 Hz, equivalent to 8 reversals per second), a reversal rate of 2 Hz (4 reversals/sec), a maximum contrast for black and white squares and a photopic luminance. In a pattern electroretinogram (PERG), the wavelength indicated with the P50-N95 values, and the wave latency indicated with the P50 values are generally considered. Comparing two electroretinographic traces performed at different times, an improvement is associated with an increase in the amplitude values and a decrease in the latency values between the second and the first trace.

### Visual Evoked Potentials

Visual evoked potentials (VEPs) enable electrophysiological exploration of the functionality of the optic pathways by recording of the variations in the bioelectric potentials of the occipital cortex evoked by visual stimuli.

In the VEP pattern at 60' and 15', the wave amplitude indicated with the N75 - P100 values and the wave latency indicated with the P100 values are generally considered. In this case as well, comparing two electroretinographic traces performed at different times, an improvement is observed if the amplitude values increase and the latency values decrease between the second and the first trace.

### Measurement of retinal ganglion cell layer thickness.

Optical coherence tomography (OCT) is a non-invasive examination that uses a laser beam to measure retinal nerve fibre layer thickness in vivo; therefore, it is an effective technique for analysing neuroprotection mechanisms. In optical coherence tomography, a measurement is generally made of the mean thickness of the retinal nerve fibre layer (RNFL), which physiologically has a double hump pattern (the fibre layer thickness is greater in the *temporal* and *superior* sectors, whereas it becomes thinner in the *nasal* and *inferior* sectors)

### Patients

The clinical study was conducted on four patients undergoing treatment at the Operational Ophthalmology Unit of the *Alma Mater Studiorum* University of Bologna and the S. Orsola - Malpighi University Hospital of Bologna.

Patient 1, a 76-year-old female, had been affected by primary open-angle glaucoma (POAG) for 4 years, with good pharmacological control of intraocular pressure measured by tonometry and always within normal limits.

Patient 2, a 53-year-old male, had been affected by primary open-angle glaucoma (POAG) for 2 years, with good pharmacological control of intraocular pressure measured by tonometry and always within normal limits.

Patient 3, a 74-year-old male, had been affected by primary open-angle glaucoma (POAG) for 7 years, with good pharmacological control of intraocular pressure measured by tonometry and always within normal limits.

Patient 4, a 74-year-old male, had been affected by primary open-angle glaucoma (POAG) for 11 years, with good pharmacological control of intraocular pressure measured by tonometry and always within normal limits.

### Treatment and results

Patient 1 (Pz1) started the treatment in March 2018 and was treated for 2 months with 2 lots of cord blood serum-based collyrium, prepared as per example 4.1; each lot included 30 vials, for a total of 60 administrations. Every vial contained the amount of collyrium necessary for one day of treatment, for both eyes: the entire content was administered within 12 hours after the vial had been opened and the vial was stored at a temperature comprised between 4°C and 10°C between one administration and another. The collyrium was administered 8 times a day, approximately every 2 hours, from the time of awakening to before bedtime, by instilling 1 drop in each eye at every administration. The HFA 30-2 analysis performed in the two years prior to the start of treatment had revealed a distinct reduction in the MD value and progressive worsening thereof, above all in the right eye (OD). In particular, in the period from December 2016 to September 2017, the MD value of visual field sensitivity went from -5.55 to -7.39 in the right eye (OD) versus -5.31 to -5.82 in the left eye (OS).

For the present analysis, use was made of the HFA 24-2 and central 10-2 programs with a finer grid model in order to improve the resolution of the remaining visual field.

Table 1 below shows the results of the analysis obtained in the period March-May 2018. It is possible to observe a progressive improvement in the MD values in terms of the visual field defect mainly in the right eye, which was the "worse" of the two. This period coincides with the treatment with the cord blood serum-based collyrium of the present invention.

**Table 1**

| Pz1 | IOP (mm/Hg) | OCT RNFL | Visual field 24-2, MD | Visual field 10-2, MD | PSD 24-2, (dB) | PSD 10-2, (dB) | PERG P50 latency (ms) | PERG P50-N95 amplitude (µV) | VEP 60' P100 latency (ms | VEP 60' N75-100 amplitude (µV) | VEP 15' P100 latency, (ms) | VEP 15' N75-100 amplitude, (µV) |
|---|---|---|---|---|---|---|---|---|---|---|---|---|
| Before treatment | | | | | | | | | | | | |
| OD | 12 | 57 | -12.72 | -17.85 | 7.36 | 7.86 | 49.8 | 2.66 | 93.75 | 4.69 | 117.77 | 216 |
| OS | 16 | 54 | -7.79 | -12.45 | 5.79 | 7.16 | 59.08 | 1.32 | 98.44 | 5.99 | 124.22 | 1.27 |

| After treatment | | | | | | | | | | | | |
|---|---|---|---|---|---|---|---|---|---|---|---|---|
| OD | 15 | 63 | -12.13 | -16.54 | 7.91 | 7.15 | 53.22 | 1.98 | 108.98 | 5.54 | 114.84 | 152 |
| OS | 18 | 60 | -8.34 | -12.56 | 4.98 | 7.37 | 57.62 | 5.31 | 93.75 | 5.6 | 115.43 | 4.54 |

It is also possible to note that the RNFL thickness values increased following treatment in both eyes. Furthermore, an improvement in the PERG and VEP 60' and 15' values was observed in the left eye, which, from a visual field standpoint, was the "better" of the two.

Patient 2 (Pz2) started the treatment in April 2018 and was treated for 2 months with 2 lots of cord blood serum-based collyrium, prepared as per example 4.1; each lot included 30 vials, for a total of 60 administrations. Every vial contained the amount of collyrium necessary for one day of treatment, for both eyes: the entire content was administered within 12 hours after the vial had been opened and the vial was stored at a temperature comprised between 4°C and 10°C between one administration and another. The collyrium was administered 8 times a day, approximately every 2 hours, from the time of awakening to before bedtime, by instilling 1 drop in each eye at every administration.

The HFA 30-2 analysis performed in the two years prior to the start of treatment had revealed a great reduction in the MD value and a progressive worsening thereof in both eyes. In particular, in the period from January 2017 to October 2017, the MD value of visual field sensitivity went from -4.0 to -6.73 in the right eye (OD) versus -2.87 to -3.63 in the left eye (OS).

For the present analysis, use was made of the HFA 24-2 and central 10-2 programs with a finer grid model in order to improve the resolution of the remaining visual field. Table 2 below shows the results of the analysis obtained in the period April-June 2018. It is possible to observe a progressive improvement in the of MD and VEP 60' and 15' values mainly in the right eye, which was the "worse" of the two. This period coincides with the treatment with the cord blood serum-based collyrium.

**Table 2**

| Pz2 | IOP (mm/Hg) | OCT RNFL | Visual field 24-2, MD | Visual field 10-2, MD | PSD 24-2, (dB) | PSD 10-2, (dB) | PERG P50 latency (ms) | PERG P50-N95 amplitude (µV) | VEP 60' P100 latency (ms) | VEP 60' N75-100 amplitude (µV) | VEP 15' P100 latency, (ms) | VEP 15' N75-100 amplitude, (µV) |
|---|---|---|---|---|---|---|---|---|---|---|---|---|
| Before treatment | | | | | | | | | | | | |
| OD | 12 | 58 | -7.71 | -5.76 | 5.28 | 1.38 | 52.25 | 1.89 | 117.77 | 2.62 | 114.84 | 2.34 |
| OS | 12 | 63 | -5.00 | -3.39 | 2.51 | 1.22 | 63.96 | 1.09 | 110.74 | 4.1 | 114.84 | 3.68 |

| After treatment | | | | | | | | | | | | |
|---|---|---|---|---|---|---|---|---|---|---|---|---|
| OD | 12 | 59 | -6.77 | -5.99 | 3.19 | 1.39 | 46.39 | 1.71 | 117.77 | 3.37 | 127.15 | 3.08 |
| OS | 11 | 63 | -5.53 | -4.14 | 2.13 | 2.13 | 49.8 | 1.9 | 111.91 | 3.18 | 118.36 | 2.82 |

It is also possible to note that the RNFL thickness values remained substantially unchanged following treatment in both eyes, whereas a distinct improvement in the PERG values was observed not only in the left eye, which, in terms of the visual field defect, was the "better" of the two, but also a partial improvement in the right eye.

Patient 3 (Pz3) started the treatment in April 2018 and was treated for 2 months with 2 lots of cord blood serum-based collyrium, prepared as per example 4.1; each lot included 30 vials, for a total of 60 administrations. Every vial contained the amount of collyrium necessary for one day of treatment, for both eyes: the entire content was administered within 12 hours after the vial had been opened and the vial was stored at a temperature comprised from 4°C to 10°C between one administration and another. The collyrium was administered 8 times a day, approximately every 2 hours, from the time of awakening to before bedtime, by instilling 1 drop in each eye at every administration.

The HFA 30-2 analysis performed in the two years prior to the start of treatment had revealed a distinct reduction in the MD value and a progressive worsening thereof, in both eyes. In particular, in the period from December 2017 to March 2018, the MD value of visual field sensitivity in the right eye (OD) went from -20.03 to -22.44, versus -7.92 to -9.34 in the left eye (OS).

For the present analysis, use was made of the HFA 24-2 and central 10-2 programs with a finer grid model in order to improve the resolution of the remaining visual field.

Table 3 below shows the results of the analysis obtained in the period April-June 2018. It is possible to observe a progressive improvement in the MD values in terms of the visual field defect mainly in the right eye, which was the "worse" of the two. This period coincides with the treatment with the cord blood serum-based collyrium of the present invention.

**Table 3**

| Pz3 | IOP (mm/H g) | OCT RNF L | Visual field 24-2, MD | Visual field 10-2, MD | PSD 24-2, (dB) | PSD 10-2, (dB) | PERG P50 latency (ms) | PERG P50-N95 amplitud e (µV) | VEP 60' P100 latency (ms | VEP 60' N75-100 amplitud e (µV) | VEP 15' P100 latency, (ms) | VEP 15' N75-100 amplitude , (µv) |
|---|---|---|---|---|---|---|---|---|---|---|---|---|
| Before treatment | | | | | | | | | | | | |
| OD | 11 | 60 | -23.72 | -21.24 | 10.86 | 8.67 | 51.27 | 3.48 | 119.53 | 4.37 | 125.39 | 3.47 |
| OS | 13 | 62 | -7.92 | -13.62 | 7.76 | 10.55 | 45.9 | 3.52 | 117.77 | 10.18 | 118.95 | 5.92 |

| After treatment | | | | | | | | | | | | |
|---|---|---|---|---|---|---|---|---|---|---|---|---|
| OD | 12 | / | -22.44 | -19.95 | 10.66 | 10.67 | 44.92 | 3.71 | 128.91 | 3.48 | 128.91 | 3.48 |
| OS | 13 | / | -9.34 | -14.04 | 8.68 | 10.91 | 44.92 | 3.15 | 110.74 | 9.97 | 108.98 | 998 |

It is also possible to note that the RNFL thickness values remained substantially unchanged following treatment in both eyes, whereas a distinct improvement in the PERG and VEP values was observed in the left eye, which, in terms of the visual field defect, was the "better" of the two, but also a partial improvement in the right eye.

Patient 4 (Pz4) started the treatment in April 2018 and was treated for 2 months with 2 lots of cord blood serum-based collyrium, prepared as per example 4.1; each lot included 30 vials, for a total of 60 administrations. Every vial contained the amount of collyrium necessary for one day of treatment, for both eyes: the entire content was administered within 12 hours after the vial had been opened and the vial was stored at a temperature comprised between 4°C and 10°C between one administration and another. The collyrium was administered 8 times a day, approximately every 2 hours, from the time of awakening to before bedtime, by instilling 1 drop in each eye at every administration.

The HFA 30-2 analysis performed in the two years prior to the start of treatment had revealed a distinct reduction in the MD value and a progressive worsening thereof, above all in the left eye. In particular, in the period from January 2014 to March 2018, the MD value of visual field sensitivity went from -0.33 to -0.59 in the right eye (OD), versus -2.95 to -9.33 in the left eye (OS).

For the present analysis, use was made of the HFA 24-2 and central 10-2 programs with a finer grid model in order to improve the resolution of the remaining visual field.

Table 4 below shows the results of the analysis obtained in the period April-June 2018. It is possible to observe a modest improvement in the MD values solely in the central portion compared to the baseline, in both eyes. This period coincides with the treatment with the cord blood serum-based collyrium of the present invention.

**Table 4**

| Pz4 | IOP (mm/Hg) | OCT RNFL | Visual field 24-2, MD | Visual field 10-2, MD | PSD 24-2, (dB) | PSD 10-2, (dB) | PERG P50 latency (ms) | PERG P50-N95 amplitude (µV) | VEP 60' P100 latency (ms | VEP 60' N75-100 amplitude (µV) | VEP 15' P100 latency, (ms) | VEP 15' N75-100 amplitude, (µV) |
|---|---|---|---|---|---|---|---|---|---|---|---|---|
| Before treatment | | | | | | | | | | | | |
| OD | 9 | 89 | -0.4 | -0.21 | 1.78 | 1.65 | 47.85 | 3.67 | 105.45 | 10.16 | 133.01 | 12.23 |
| OS | 15 | 56 | -7.09 | -6.13 | 3.55 | 1.87 | 48.83 | 2.23 | 117.77 | 10.07 | 149.41 | 6.51 |

| After treatment | | | | | | | | | | | | |
|---|---|---|---|---|---|---|---|---|---|---|---|---|
| OD | 14 | 90 | -1.06 | -0.12 | 2.22 | 1.24 | 49.8 | 2.75 | 109.57 | 18.18 | 124.22 | 17.43 |
| OS | 17 | 56 | -9.00 | -6.00 | 5.25 | 3.01 | 51.27 | 1.25 | 117.77 | 8.47 | 143.55 | 10.72 |

It is also possible to note that the RNFL thickness values remained substantially unchanged following treatment in both eyes, whereas a distinct improvement in the PERG and VEP values was observed in both eyes, and above all, in the left eye, which, in terms of the visual field defect, was the "better" of the two. It was also possible to observe a partial improvement in the right eye.

## Claims

1. Blood serum for use in the treatment of neurodegenerative ophthalmologic pathologies, wherein said serum comprises the nerve growth factor-β (NGF-β), wherein said neurodegenerative ophthalmologic pathologies are selected from the group consisting of: degenerative pathologies of the retina, degenerative pathologies of the optic nerve, and ophthalmologic pathologies whose progression is linked to the activation of neuroinflammatory processes.

2. The serum for use according to claim 1, wherein said nerve growth factor-β (NGF-β) is present in a concentration equal to or greater than 5 pg/ml.

3. The serum for use according to claim 1, comprising the nerve growth factor-β (NGF-β) in a concentration equal to or greater than 6 pg/ml, preferably in a concentration equal to or greater than 7 pg/ml, more preferably in a concentration equal to or greater than 8 pg/ml.

4. The serum for use according to claim 1, comprising the nerve growth factor-β (NGF-β) in a concentration equal to or less than 5 pg/ml, preferably comprised between 0.5 and 5 pg/ml, advantageously comprised from 0.7 to 4 pg/ml.

5. The serum for use according to claim 1, further comprising the brain-derived neurotrophic factor (BDNF), preferably in a concentration equal to or greater than 9500 pg/ml.

6. The serum for use according to any one of claims 1 to 5, further comprising one or more molecules selected from the group consisting of:
- epidermal growth factor (EGF), preferably in a concentration equal to or greater than 500 pg/ml;
- transforming growth factor-α (TGF-α), preferably in a concentration equal to or greater than 50 pg/ml or equal to or less than 50 pg/ml
- platelet-derived growth factor (PDGF), preferably in a concentration equal to or greater than 5000 pg/ml;
- fibroblast growth factor (FGF), preferably in a concentration equal to or greater than 200 pg/ml;
- interleukin 10 (IL-10), preferably in a concentration equal to or greater than 5 pg/ml;
- interleukin 13 (IL-13), preferably in a concentration equal to or greater than 100 pg/ml.
- glial cell-line derived neurotrophic factor (GDNF), preferably in a concentration equal to or greater than 0.5 pg/ml.

7. The serum for use according to any one of the preceding claims, wherein said serum is serum from umbilical cord blood.

8. The serum for use according to any one of the preceding claims, wherein said serum is serum from human blood.

9. The serum for use according to claim 1, wherein the neurodegenerative ophthalmologic pathology is glaucoma.

10. The serum for use according to claim 1, wherein the ophthalmologic pathology whose progression is linked to the activation of neuroinflammatory processes is selected from among: retinitis pigmentosa, age-related macular degeneration (AMD), diabetic retinopathy or Usher syndrome.

11. Method for preparing the serum for use according to any one of claims 1-10, comprising the steps of:
- analysing the NGF-β concentration in serum samples obtained from corresponding blood samples; and
- selecting the serum samples having a NGF-β concentration of equal to or greater than 5 pg/ml.

12. The method according to claim 11, comprising the steps of:
i. analysing the BDNF concentration in serum samples obtained from corresponding blood samples; and
ii. selecting the serum samples having a BDNF concentration equal to or greater than 9500 pg/ml.

13. The method according to claim 11 or 12, wherein said one or more blood samples are blood samples from a human umbilical cord.

14. Composition comprising the serum for use according to any one of claims 1-10 and, optionally, one or more excipients and/or carriers and/or preservatives acceptable for human use.

15. The composition for use according to claim 14, wherein said one or more excipients are an aqueous physiological solution.

16. The composition for use according to claim 14 or claim 15, wherein said composition is in a form selected from the group consisting of: collyrium, eye or nasal drops, eye or nasal ointment, eye or nasal gel, or nasal spray.

## Patentansprüche

1. Blutserum zur Verwendung bei der Behandlung von neurodegenerativen ophthalmologischen Pathologien, wobei das Serum den Nervenwachstumsfaktor-β (NGF-β) umfasst, wobei die neurodegenerativen ophthalmologischen Pathologien aus der Gruppe ausgewählt sind, die besteht aus: degenerativen Pathologien der Netzhaut, degenerativen Pathologien des Sehnervs und ophthalmologischen Pathologien, deren Progression mit der Aktivierung von neuroinflammatorischen Prozessen verbunden ist.

2. Serum zur Verwendung nach Anspruch 1, wobei der Nervenwachstumsfaktor-β (NGF-β) in einer Konzentration gleich oder größer als 5 pg/ml vorliegt.

3. Serum zur Verwendung nach Anspruch 1, umfassend den Nervenwachstumsfaktor-β (NGF-β) in einer Konzentration gleich oder größer als 6 pg/ml, vorzugsweise in einer Konzentration gleich oder größer als 7 pg/ml, bevorzugter in einer Konzentration gleich oder größer als 8 pg/ml.

4. Serum zur Verwendung nach Anspruch 1, umfassend den Nervenwachstumsfaktor-β (NGF-β) in einer Konzentration gleich oder weniger als 5 pg/ml, vorzugsweise zwischen 0,5 und 5 pg/ml, vorteilhafterweise von 0,7 bis 4 pg/ml.

5. Serum zur Verwendung nach Anspruch 1, ferner umfassend den vom Gehirn abgeleiteten neurotrophen Faktor (BDNF), vorzugsweise in einer Konzentration gleich oder größer als 9500 pg/ml.

6. Serum zur Verwendung nach einem der Ansprüche 1 bis 5, ferner umfassend ein oder mehrere Moleküle, ausgewählt aus der Gruppe, bestehend aus:
- epidermalem Wachstumsfaktor (EGF), vorzugsweise in einer Konzentration gleich oder größer als 500 pg/ml;
- transformierendem Wachstumsfaktor-α (TGF-α), vorzugsweise in einer Konzentration gleich oder größer als 50 pg/ml oder gleich oder weniger als 50 pg/ml;
- von Blutplättchen abgeleitetem Wachstumsfaktor (PDGF), vorzugsweise in einer Konzentration gleich oder größer als 5000 pg/ml;
- Fibroblasten-Wachstumsfaktor (FGF), vorzugsweise in einer Konzentration gleich oder größer als 200 pg/ml;
- Interleukin 10 (IL-10), vorzugsweise in einer Konzentration gleich oder größer als 5 pg/ml;
- Interleukin 13 (IL-13), vorzugsweise in einer Konzentration gleich oder größer als 100 pg/ml;
- Gliazelllinien-abgeleitetem neurotrophem Faktor (GDNF), vorzugsweise in einer Konzentration gleich oder größer als 0,5 pg/ml.

7. Serum zur Verwendung nach einem der vorhergehenden Ansprüche, wobei es sich beim Serum um Serum aus Nabelschnurblut handelt.

8. Serum zur Verwendung nach einem der vorhergehenden Ansprüche, wobei es sich beim Serum um Serum aus menschlichem Blut handelt.

9. Serum zur Verwendung nach Anspruch 1, wobei die neurodegenerative ophthalmologische Pathologie Glaukom ist.

10. Serum zur Verwendung nach Anspruch 1, wobei die ophthalmologische Pathologie, deren Progression mit der Aktivierung von neuroinflammatorischen Prozessen verbunden ist, ausgewählt ist aus: Retinitis pigmentosa, altersbedingter Makuladegeneration (AMD), diabetischer Retinopathie oder Usher-Syndrom.

11. Verfahren zur Herstellung des Serums zur Verwendung nach einem der Ansprüche 1-10, umfassend die Schritte:
- Analysieren der NGF-β-Konzentration in Serumproben, die aus entsprechenden Blutproben gewonnen wurden; und
- Auswählen der Serumproben mit einer NGF-β-Konzentration gleich oder größer als 5 pg/ml.

12. Verfahren nach Anspruch 11, umfassend die folgenden Schritte:
i. Analysieren der BDNF-Konzentration in Serumproben, die aus entsprechenden Blutproben gewonnen wurden, und
ii. Auswählen der Serumproben mit einer BDNF-Konzentration gleich oder größer als 9500 pg/ml.

13. Verfahren nach Anspruch 11 oder 12, wobei es sich bei der einen oder mehreren Blutproben um Blutproben aus einer menschlichen Nabelschnur handelt.

14. Zusammensetzung, umfassend das Serum zur Verwendung nach einem der Ansprüche 1-10 und gegebenenfalls einen oder mehrere Exzipienten und/oder Träger und/oder Konservierungsmittel, die für den menschlichen Gebrauch annehmbar sind.

15. Zusammensetzung zur Verwendung nach Anspruch 14, wobei es sich bei dem einen oder mehreren Exzipienten um eine wässrige physiologische Lösung handelt.

16. Zusammensetzung zur Verwendung nach Anspruch 14 oder Anspruch 15, wobei die Zusammensetzung in einer Form vorliegt, die aus der Gruppe ausgewählt ist, die besteht aus: Collyrium, Augen- oder Nasentropfen, Augen- oder Nasensalbe, Augen- oder Nasengel oder Nasenspray.

## Revendications

1. Sérum de sang utilisé dans le traitement de pathologies ophtalmologiques neurodégénératives, dans lequel ledit sérum comprend le facteur de croissance nerveuse β (NGF-β), dans lequel lesdites pathologies ophtalmologiques neurodégénératives sont choisies dans le groupe constitué par : les pathologies dégénératives de la rétine, les pathologies dégénératives du nerf optique, et les pathologies ophtalmologiques dont la progression est liée à l'activation de processus neuroinflammatoires.

2. Sérum utilisé selon la revendication 1, dans lequel ledit facteur de croissance nerveuse β (NGF-β) est présent à une concentration supérieure ou égale à 5 pg/ml.

3. Sérum utilisé selon la revendication 1, comprenant le facteur de croissance nerveuse β (NGF-β) à une concentration supérieure ou égale à 6 pg/ml, de préférence à une concentration supérieure ou égale à 7 pg/ml, plus préférablement à une concentration supérieure ou égale à 8 pg/ml.

4. Sérum utilisé selon la revendication 1, comprenant le facteur de croissance nerveuse β (NGF-β) à une concentration inférieure ou égale à 5 pg/ml, de préférence comprise entre 0,5 et 5 pg/ml, avantageusement comprise entre 0,7 et 4 pg/ml.

5. Sérum utilisé selon la revendication 1, comprenant de plus le facteur neurotrophique dérivé du cerveau (BDNF), de préférence à une concentration supérieure ou égale à 9 500 pg/ml.

6. Sérum utilisé selon l'une quelconque des revendications 1 à 5, comprenant de plus une ou plusieurs molécules choisies dans le groupe constitué par :
- le facteur de croissance épidermique (EGF), de préférence à une concentration supérieure ou égale à 500 pg/ml ;
- le facteur de croissance transformant α (TGF-α), de préférence à une concentration supérieure ou égale à 50 pg/ml ou inférieure ou égale à 50 pg/ml ;
- le facteur de croissance dérivé des plaquettes (PDGF), de préférence à une concentration supérieure ou égale à 5 000 pg/ml ;
- le facteur de croissance des fibroblastes (FGF), de préférence à une concentration supérieure ou égale à 200 pg/ml ;
- l'interleukine 10 (IL-10), de préférence à une concentration supérieure ou égale à 5 pg/ml ;
- l'interleukine 13 (IL-13), de préférence à une concentration supérieure ou égale à 100 pg/ml ;
- le facteur neurotrophique dérivé des cellules gliales (GDNF), de préférence à une concentration supérieure ou égale à 0,5 pg/ml.

7. Sérum utilisé selon l'une quelconque des revendications précédentes, dans lequel ledit sérum est un sérum provenant de sang de cordon ombilical.

8. Sérum utilisé selon l'une quelconque des revendications précédentes, dans lequel ledit sérum est un sérum provenant de sang humain.

9. Sérum utilisé selon la revendication 1, dans lequel la pathologie ophtalmologique neurodégénérative est le glaucome.

10. Sérum utilisé selon la revendication 1, dans lequel la pathologie ophtalmologique dont la progression est liée à l'activation de processus neuroinflammatoires est choisie parmi : la rétinite pigmentaire, la dégénérescence maculaire liée à l'âge (DMLA), la rétinopathie diabétique ou le syndrome d'Usher.

11. Procédé de préparation du sérum utilisé selon l'une quelconque des revendications 1-10, comprenant les étapes de :
- analyser la concentration de NGF-β dans les échantillons de sérum obtenus à partir des échantillons de sang correspondants ; et
- choisir les échantillons de sérum ayant une concentration en NGF-β supérieure ou égale à 5 pg/ml.

12. Procédé selon la revendication 11, comprenant les étapes de :
i. analyser la concentration en BDNF dans les échantillons de sérum obtenus à partir des échantillons de sang correspondants ; et
ii. choisir les échantillons de sérum ayant une concentration en BDNF supérieure ou égale à 9 500 pg/ml.

13. Procédé selon la revendication 11 ou 12, dans lequel lesdits un ou plusieurs échantillons de sang sont des échantillons de sang provenant d'un cordon ombilical humain.

14. Composition, comprenant le sérum utilisé selon l'une quelconque des revendications 1-10 et, éventuellement, un ou plusieurs excipients et/ou vecteurs et/ou conservateurs acceptables pour une utilisation humaine.

15. Composition utilisée selon la revendication 14, dans laquelle lesdits un ou plusieurs excipients sont une solution physiologique aqueuse.

16. Composition utilisée selon la revendication 14 ou la revendication 15, dans laquelle ladite composition est sous une forme choisie dans le groupe constitué par : le collyre, les gouttes oculaires ou nasales, la pommade oculaire ou nasale, le gel oculaire ou nasal, ou le spray nasal.
